# EUROPEAN PATENT APPLICATION

(11) **EP 4 449 980 A1**
(43) Date of publication of application: **23.10.2024**
(21) Application number: 23168915.9
(22) Date of filing: 20.04.2023
(51) Int. Cl.: A61B 5/0205, A61B 5/024, A61B 5/00, G16H 50/20, G16H 50/30, A61B 5/11

(54) **COMPUTER-IMPLEMENTED METHOD, APPARATUS, COMPUTER PROGRAM PRODUCT, AND COMPUTER-READABLE STORAGE MEDIUM**

(71) Applicant: Koninklijke Philips N.V., 5656 AG Eindhoven (NL)
(72) Inventor: WULTERKENS, Bernice Marielouise, Eindhoven (NL); DOS SANTOS DA FONSECA, Pedro Miguel Ferreira, Eindhoven (NL); HERMANS, Lieke Wilhelmina Alexandra, 5656AG Eindhoven (NL); VAN GILST, Merel, Eindhoven (NL); OVEREEM, Sebastiaan, Eindhoven (NL)
(74) Representative: Philips Intellectual Property & Standards

(57) **Abstract**

There is provided a computer-implemented method comprising the steps of: receiving an arousal signal representative of an occurrence an arousal of a subject; receiving a heartrate signal representative of a heartrate of the subject; determining a recovery response phase signal in the heartrate signal; wherein the recovery response phase signal is representative of a recovery response phase in which the heartrate decreases after the arousal; determining a deviation of the recovery response phase signal relative to a reference signal. Further, there is provided an apparatus comprising a sensor system and a processing unit coupled to the sensor system. The sensor system is configured to provide a heartrate signal representative of a heartrate of a subject. The sensor system is configured to provide an arousal signal representative of an occurrence of an arousal of a subject. The processing unit is configured to perform the computer-implemented method.

## Description

### FIELD OF THE INVENTION

The invention relates to a computer-implemented method, an apparatus, a computer program product, and a computer-readable storage medium.

### BACKGROUND OF THE INVENTION

Insomnia and obstructive sleep apnoea (OSA) commonly co-occur, known as comorbid insomnia and obstructive sleep apnoea (COMISA). COMISA is associated with greater morbidity for a subject compared to insomnia or OSA alone. Differentiating between insomnia, OSA and COMISA is challenging due to the overlapping symptoms associated with insomnia and OSA and due to the different approaches used to identify each disorder. For instance, while polysomnography is considered by some to be the gold standard technique for identifying OSA, the identification of insomnia is typically based on subjective measurements. Single night standard sleep parameters derived from single night polysomnography are not sufficient to distinguish between subjects with COMISA and subjects with only OSA. For a subject suffering from COMISA, once insomnia or OSA has been identified, the other condition can be difficult to identify and is often missed. Identifying that a subject is suffering from COMISA is important as appropriate treatment options can then be offered. For example, subjects with COMISA may benefit from cognitive behavioral therapy for insomnia (e.g., CBTi) before, or at the same time as, starting positive airway pressure (PAP) therapy. A subject with COMISA who only uses PAP therapy will likely fail adherence criteria, and may even drop out of PAP therapy altogether.

### SUMMARY OF THE INVENTION

It is an objective of the invention to provide an improved way to determine whether a subject has OSA or COMISA. Optionally, the improved way is based on measurements on the subject that are obtained in a non-obtrusive way.

According to a first specific aspect, there is provided a computer-implemented method comprising:
receiving an arousal signal representative of an occurrence an arousal of a subject;
receiving a heartrate signal representative of a heartrate of the subject;
determining a recovery response phase signal in the heartrate signal,
wherein the recovery response phase signal is representative of a recovery response phase in which the heartrate decreases after the arousal;
determining a deviation of the recovery response phase signal relative to a reference signal.

Both OSA and insomnia are associated with arousals. Arousals lead to an increase in blood pressure and heart rate. The inventors have discovered that the recovery response phase of the heart rate after an arousal occurs, is slower in the presence of insomnia. This insight is used to identify the possible presence of comorbid insomnia in patients with OSA. In case the recovery response phase signal compared to the reference signal is normal or similar to that of a subject having OSA, it can be determined the subject does not have insomnia. In case the recovery response signal compared to the reference signal is slow, it can be determined that the subject has insomnia.

The arousal signal is a signal that indicates that an arousal has occurred. For example, the arousal signal is generated by a sensor. The sensor is for example part of a measurement device located at a sleep center, such polysomnography apparatus. The sensor is for example a wearable sensor, such as a wearable EEG sensor. For example, the sensor is a cardiac sensor, such as ECG, PPG, or BCG. For example, the sensor is a respiratory sensor, such as a respiratory belt, airflow sensor, bed sensor, or Doppler radar installed on the night table. For example, the sensor is a motion sensor for detecting a motion of the subject. A movement of the subject indicates the occurrence of an arousal. For example, the sensor is a sound sensor, such as a microphone. Based on the sound of the breathing of the subject, the occurrence of an arousal is detected. The arousal signal is, for example, a signal that is received directly from a sensor. In another example, the arousal signal is received from a pre-processing unit. The pre-processing unit is configured to receive a sensor signal from the sensor. The pre-processing unit is configured to process the sensor signal to generate the arousal signal. For example, the pre-processing unit is configured to determine whether an occurrence of an arousal is present in the sensor signal. The arousal signal is, for example, a signal representing a parameter of the subject over time. In another example, the arousal signal is a binary signal that indicates whether the arousal is occurring or is not occurring.

The heartrate signal is a signal that is representative of the heartrate of the subject. For example, the heartrate signal is generated by a cardiac sensor. The sensor is, for example, part of a heartrate measurement device located at a sleep center, such as an electrocardiography (ECG) apparatus or a ballistocardiography sensor. The heartrate sensor is for example a wearable sensor, such as a wearable ECG sensor or a photoplethysmogram (PPG) sensor. The wearable sensor is, for example, arranged to be worn by the subject at the wrist or on the chest or on the head or on a finger of the subject. For example, the sensor is placed on or under the mattress of the bed, such as a BCG sensor. The heartrate signal is, for example, a signal that is received directly from a sensor. In another example, the heartrate signal is received from a pre-processing unit. The pre-processing unit is configured to receive a sensor signal from the heartrate sensor. The pre-processing unit is configured to process the sensor signal to generate the heartrate signal. For example, the pre-processing unit is configured to determine from the sensor signal time intervals between heartbeats and/or timings of heart beats. The heartrate signal is, for example, a signal representing the instant heart rate and/or heart rate variability.

The occurrence of an arousal causes the heartrate of the subject to increase. The heartrate increases from a baseline value. The baseline value is the heartrate prior to the arousal. For example, the baseline value is an average heartrate of the subject while sleeping and without having an arousal. Due to the arousal, the heartrate increases to a maximum heartrate value. After the maximum heartrate value, the heart rate decreases. The recovery response phase represents the phase in which the heart rate decreases after the maximum heartrate value. For example, the recovery response phase starts when the heart is at the maximum heartrate value and ends when the heartrate is back at the baseline value or is substantially back at the baseline value. Substantially back at the baseline value is, for example, less than 10% higher than the baseline value, or less than 5% or less than 3%. The recovery response phase signal comprises, for example, the entire recovery response phase or only part of the recovery response phase. For example, the recovery response phase signal comprises a part of the recovery response near the maximum heartrate value. For example, the recovery response phase signal comprises a part of the recovery response near the end of the recovery response phase. It is noted that the maximum heartrate value is a local maximum in temporal vicinity of when an arousal occurs. It is possible that the subject has a higher heartrate during different sleep stages or during awakenings.

It is noted that the situation may occur that during the recovery response phase, the subject causes the heartrate to increase, for example by moving or by waking up. In this situation, the recovery response phase does not proceed all the way to the end at the baseline value. In this situation, the computer-implemented method is, for example, able to determine the recovery response phase signal based on the heartrate signal of the partial recovery response phase. In another example, the computer-implemented method is not able to determine the recovery response phase signal based on the heartrate signal of the partial recovery response phase. In this example, the computer-implemented method is able to determine the deviation when the next arousal signal is received. In another example, during the recovery response phase, another arousal occurs, causing the heartrate to increase again. In this example, the computer-implemented method determines the recovery response phase signal after the other arousal.

The recovery response signal is compared with a reference signal. The reference signal is for example a certain heartrate value after a certain amount of time after the arousal has occurred. For example, the reference signal is a heartrate value that is 2 or 4 or 6 beats per minute lower than the maximum heartbeat value. The certain amount of time is, for example, 20 seconds or 30 seconds of 45 seconds or 1 minute. In case the recovery response signal indicates that the heartrate at the certain amount of time is higher than the reference signal, this may provide information about the likeliness that the subject has COMISA or insomnia. In case the recovery response signal indicates that the heartrate at the certain amount of time is lower than or equal to the reference signal, this may provide information about the likeliness that the subject does not have COMISA and does not have insomnia. For example, the reference signal represents a gradient of the decrease of the heartrate in the recovery response phase. In case the gradient of the recovery response signal is lower than the gradient of the reference signal, this may provide information about the likeliness that the subject has COMISA or insomnia. In case the gradient of the recovery response signal is higher than the gradient of the reference signal, this may provide information about the likeliness that the subject does not have COMISA and does not have insomnia. The reference signal represents, for example, the recovery response phase of a healthy person. The reference signal represents, for example, the recovery response phase of a person having a condition and/or an illness.

For example, the deviation is communicated to a user interface, for example via Wi-Fi or Bluetooth or any other type of wired or wireless communication method. The subject and/or the healthcare professional is informed about the deviation. Based on the deviation, the healthcare professional is, for example, able to decide on the best treatment for the subject.

In an embodiment, the computer-implemented method comprises receiving the arousal signal from an arousal sensor, and receiving the heartrate signal from a cardiac sensor. The arousal sensor is configured to provide the arousal signal representative of the occurrence of the arousal. The cardiac sensor is configured to provide the heartrate signal representative of the heart rate.

According to this embodiment, the arousal signal is provided by an arousal sensor, and the heartrate sensor is provided by a heartrate sensor. Examples of the arousal sensor and the heartrate sensor have been mentioned in the text above. This embodiment has the advantage that a dedicated sensor is used to determine the occurrence of the arousal, resulting in an accurate determination of the occurrence of the arousal. Using a dedicated sensor to determine the heartrate results in an accurate determination of the heartrate. As a result, the determination of the deviation is made with improved accuracy.

In an embodiment, the computer-implemented method comprises receiving the arousal signal from a cardiac sensor, and receiving the heartrate signal from the cardiac sensor. The cardiac sensor is configured to provide the arousal signal representative of the occurrence of the arousal. The cardiac sensor is configured to provide the heartrate signal representative of the heart rate.

According to this embodiment, both the arousal signal and the heartrate signal are provided by the same sensor, i.e., the cardiac sensor. The cardiac sensor is able to obtain information about the heartrate to generate the heartrate signal. In addition the cardiac sensor is able to obtain information about the occurrence of an arousal based on the information about the heartrate or heart rate variability. Examples on how to obtain information about the occurrence of an arousal based on the information about the heartrate are published in literature, such as "An ECG-based algorithm for the automatic identification of autonomic activations associated with cortical arousal" by Basner, M., Griefahn, B., Müller, U., Plath, G. & Samel, A. Sleep 30, 1349-61 (2007), or such as "Automatic, electrocardiographic-based detection of autonomic arousals and their association with cortical arousals, leg movements, and respiratory events in sleep", by Olsen, M. et al, Sleep 41, (2018). By using the cardiac sensor for both the arousal signal and the heartrate signal, to costs of an additional sensor is saved. Also, the arousal signal and the heartrate signal are obtained in a less obtrusive way for the subject, because less sensors need to be place on the subject.

In an embodiment, the computer-implemented method comprises determining a peak response phase signal in the heartrate signal. The peak response phase signal is representative of a peak response phase in which the heartrate increases after the arousal. The recovery response phase signal starts after the peak response phase signal. For example, the recovery response phase signal starts consecutively after the peak response phase signal.

According to this embodiment, the peak response phase signal in the heartrate signal represents the increase of the heartrate caused by the arousal till the maximum heartrate value is reached. After the peak response phase signal, the heartrate decreases. The recovery response phase signal is obtained in the recovery response phase in which the heartrate decreases. As the peak response phase signal is a prominent feature in the heartrate signal, the computer-implemented method is able to detect easily when to obtain the recovery response phase signal.

In an embodiment, the peak response phase signal represents a number of cardiac cycles in the range of 2-12 after the arousal, wherein the recovery response phase signal represents a number of cardiac cycles in the range of 5-25 after an end of the peak response phase.

The inventors have discovered that the heartrate increases a number of cardiac cycles in the range of 2-12. After that, in the next 5-25 cardiac cycles, the heartrate decreases. After the 5-25 cardiac cycles, the heartrate has substantially returned to the baseline value. For example, the duration between the onset of the arousal and the end of the recovery response phase is less than 2 minutes, for example less than 1 minute, for example approximately 30 seconds.

In an embodiment, determining the deviation comprises determining the deviation, in response to receiving an SDB-signal representative of an occurrence of a Sleep Disorder Breathing (SDB) event of the subject.

According to this embodiment, an SDB-signal is provided that indicates an occurrence of an SDB-event. An SDB-event is, for example, an OSA event in which the breathing is temporarily stopped. An SDB-event is, for example, a hypopnea event in which breathing is substantially reduced, causing a significant oxygen desaturation in the blood. In many cases, an SDB-event causes an arousal. The arousal causes the increase in heartrate. By determining the deviation between the recovery response phase signal after the SDB-event with the reference signal, insight is obtained about the subject, such whether the subject is likely suffering from OSA or COMISA. Alternatively, insight is obtained about the subject by determining the deviation causes by arousals when no SDB signal was provided. This way insight is obtained about arousals that were not caused by an SDB event. The SDB-signal is, for example, provided by a Positive Airway Pressure (PAP-) device. PAP-devices are commonly used to treat sleep apnea or sleep hypopnea. The PAP-device has, for example, a sensor, such as an airflow sensor or a pressure sensor, to detect whether an SDB-event occurs. The computer-implemented method uses the signal from that sensor as the SDB-signal. In another example, a sensor is provided to generate the SDB-signal representative of an SDB-event. The sensor is, for example, an airflow sensor or a pressure sensor or a sound sensor or a respiratory sensor. For example, the SDB-signal is generated by the cardiac sensor that also generates the heartrate signal.

In an embodiment, determining the deviation comprises determining the deviation of the recovery response signal relative to a further reference signal, in response to receiving a non-SDB signal representative of an absence of a Sleep Disorder Breathing (SDB) event of the subject.

According to this embodiment, there are two reference signals, i.e., the reference signal and the further reference signal. In case the SDB-signal is received, the deviation is determined based on the reference signal, whereas in case the non-SDB-signal is received, the deviation is determined based on the further reference signal. The inventors have discovered that the recovery response phase is different for arousals caused by an SDB-event than for arousals not caused by an SDB-event. By having two different reference signals, a more accurate insight on the subject is obtained.

In an embodiment, the reference signal represents a recovery response phase of a subject having one of Obstructive Sleep Apnea (OSA), Co-Morbid Insomnia and Sleep Apnea (COMISA) and insomnia.

OSA, COMISA and insomnia have overlapping symptoms, such as daytime sleepiness. In most cases, objective measurements such as gold standard PSG are used to diagnose OSA, whereas the diagnosis of insomnia is based on subjective measurements. Because of this different approach to diagnose both disorders, it is difficult to find the other disorder if one is already diagnosed. Both OSA and insomnia are associated with arousals. Arousals lead to an increase in blood pressure and heart rate, which results in a small elevation in sympathetic tone. If arousals occur frequently, the increase in sympathetic tone can become chronic, resulting added cardiovascular burden, and possibly, an increase in long-term risk of developing cardiovascular disease. By using the reference signal representing the recovery response phase of a subject having OSA, it may be accurately determined whether the subject likely has OSA or not. Similarly, by using the reference signal representing the recovery response phase of a subject having COMISA, it may be accurately determined whether the subject likely has COMISA or not. Similarly, by using the reference signal representing the recovery response phase of a subject having insomnia, it may be accurately determined whether the subject likely has insomnia or not. For example, the reference signal representing a recovery response phase of a subject having OSA or COMISA is used in response to receiving the SDB-signal representative of an occurrence of a Sleep Disorder Breathing (SDB) event of the subject. For example, the reference signal representing a recovery phase of a subject having insomnia is used in response to receiving a non-SDB signal representative of an absence of a Sleep Disorder Breathing (SDB) event of the subject.

In an embodiment, the reference signal comprises an OSA-reference signal and a COMISA-reference signal. The OSA-reference signal is representative of a subject having OSA. The COMISA-reference signal is representative of a subject having COMISA. Determining the deviation comprises determining a first difference between the recovery response signal and the OSA-reference signal, and determining the deviation comprises determining a second difference between the recovery response signal and the COMISA-reference signal.

As OSA and COMISA have overlapping symptoms, it is difficult to distinguish these two. However, by determining the deviation of the recovery response phase signal on both the OSA-reference signal and the COMISA-reference signal, it may be accurately determined which of the two reference signals is closest to the recovery response phase of the subject. As a result, it may be determined whether the subject most likely has OSA or COMISA.

In an embodiment, the reference signal represents a value of the heartrate prior to the arousal.

The heartrate prior to the arousal forms a simple, but still accurate baseline value to which the recovery response phase signal can be compared.

In an embodiment, the computer-implemented method comprises determining a pre-arousal phase signal in the heartrate signal, and determining the reference signal based on the pre-arousal phase signal. The pre-arousal phase signal is representative of the heartbeat prior to an onset of the arousal.

According to this embodiment, the reference signal represents the base line value of the heartrate before the arousal. This provides a proper reference and reduces the influence of other factors that may change the heartrate, such as a different environmental temperature, caffeine intake, or fatigue.

In an embodiment, the pre-arousal phase signal is based on a number of cardiac cycles prior to the onset of the arousal, wherein the number is in the range of 2-12.

According to this embodiment, the heartrate shortly before the arousal provides a good reference signal. By taking multiple cardiac cycles into account, measurement accuracies of the heartrate have less effect on the pre-arousal phase signal, because they are averaged out.

In a second aspect of the invention, there is provided an apparatus comprising a sensor system, and a processing unit coupled to the sensor system. The sensor system is configured to provide a heartrate signal representative of a heartrate of a subject. The sensor system is configured to provide an arousal signal representative of an occurrence of an arousal of a subject. The processing unit is configured to perform the computer-implemented method according to the first aspect of the invention.

In a third aspect of the invention, there is provided a computer program product comprising instructions which, when executed by a processing unit, cause the processing unit to carry out the computer-implemented method of the first aspect of the invention.

In a fourth aspect of the invention, there is provided a computer-readable storage medium comprising instructions which, when executed by a processing unit, cause the processing unit to carry out the computer-implemented method of the first aspect of the invention.

### BRIEF DESCRIPTION OF THE DRAWINGS

Exemplary embodiments will now be described, by way of example only, with reference to the following figures, in which:
FIG. 1 depicts an apparatus according to the second aspect of the invention;
FIG. 2 depicts a computer-implemented method according to the first aspect of the invention;
FIG. 3 depicts a change in heartrate of the subject due to any arousal;
FIG. 4 depicts a change in heartrate of the subject due to an arousal caused by an SDB event;
FIG. 5 depicts a change in heartrate of the subject due to arousals not caused by an SDB-event;
FIG. 6 depicts a processing unit executing the computer-implemented method according to the first aspect of the invention.

### DETAILED DESCRIPTION OF EMBODIMENTS

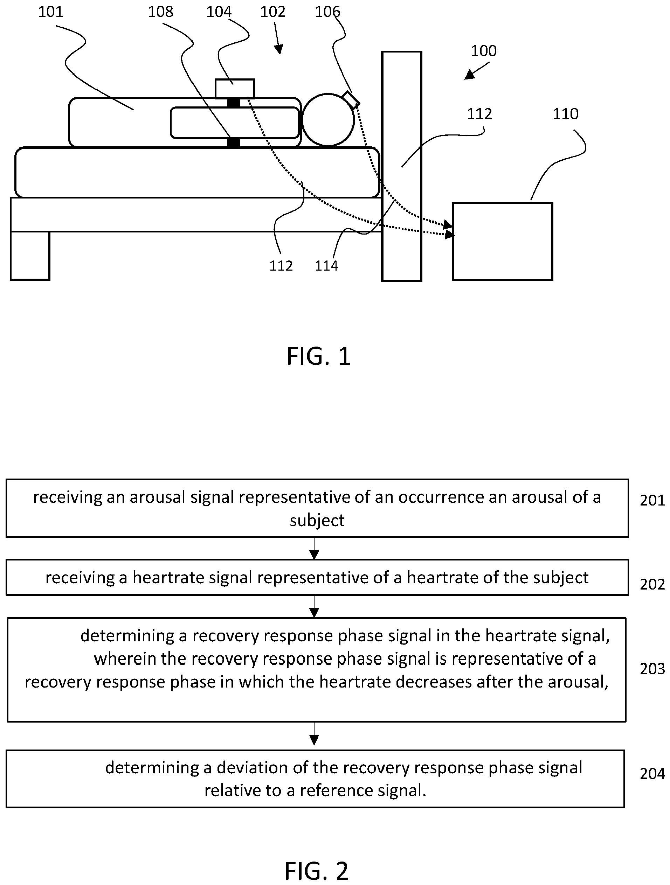

FIG. 1 depicts a first embodiment according to the invention. FIG. 1 shows an apparatus 100 comprising a sensor system 102 and a processing unit 110 coupled to the sensor system 102. The sensor system 102 is configured to provide a heartrate signal 112 representative of a heartrate of a subject 101. The sensor system 102 is configured to provide an arousal signal 114 representative of an occurrence of an arousal of the subject 101. The processing unit 110 is configured to perform the computer-implemented method as depicted in FIG 2.

The subject 101 is sleeping on a bed 112. The sensor system 102 has a cardiac sensor 104 arranged on the chest of the subject 101. The cardiac sensor 104 is attached to the subject 101 with a belt 108. The cardiac sensor 104 is configured to provide the heartrate signal 112 representative of a heartrate of a subject 101. The sensor system 102 has an EEG-sensor 106 attached to the head of the subject 101. The EEG-sensor 106 is configured to provide the arousal signal 114 representative of an occurrence of an arousal of a subject 101.

FIG. 2 depicts the computer-implemented method comprising as performed by the processing unit 110. The method comprises steps 201-204. Step 201 comprises receiving an arousal signal 114 representative of an occurrence an arousal of the subject 101. Due to the arousal, the heartrate of the subject 101 increases. Step 202 comprises receiving a heartrate signal 112 representative of a heartrate of the subject 101. Some time after the arousal, the heartrate of the subject 101 decreases in the recovery response phase. Step 203 comprises determining a recovery response phase signal 310 in the heartrate signal 112. The recovery response phase signal 310 is representative of the recovery response phase in which the heartrate decreases after the arousal. Step 204 comprises determining a deviation of the recovery response phase signal 310 relative to a reference signal.

The arousal signal 114 is received by the processing unit 110 from the arousal sensor, i.e., the EEG-sensor 106. The heartrate signal 112 is received by the processing unit 110 from a cardiac sensor 104.

In an alternative embodiment, the cardiac sensor 104 is configured to provide the arousal signal 114 representative of the occurrence of the arousal, as well as configured to provide the heartrate signal 112 representative of the heart rate.

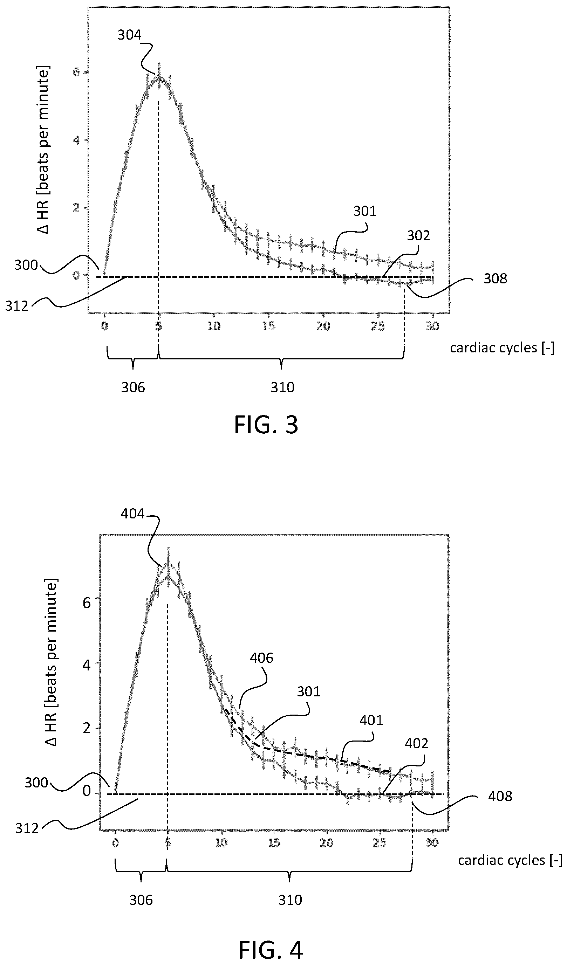

FIG. 3 depicts a change in heartrate of the subject 101 due to any arousal. The arousals are caused by an SDB-event or not caused by an SDB-event. The x-axis states the number of cardiac cycles, starting from the onset of the arousal at 300. The y-axis states the difference in heartbeats in beats per minute compared to the heartrate at the onset of the arousal at 300. Line 301 represents the averaged measured difference in heartrate of a subject with COMISA caused by any arousal. Line 302 represents the averaged measured difference in heartrate of a subject with OSA caused by any arousal.

At the onset of the arousal at 300, both lines 301 and 302 increase similarly until they reach a maximum heartrate value at 304. The maximum heartrate value at 304 is reached after 5 cardiac cycles from the onset of the arousal at 300. The maximum heartrate value at 304 is about 6 beats per minute faster than the heartrate at the onset of the arousal at 300. The peak response phase starts at the onset of the arousal and ends at the maximum heartrate value at 304. Therefore, the peak response phase signal 306, which is representative of the peak response phase in which the heartrate increases after the arousal, starts at the onset of the arousal at 300 and ends at the maximum heartrate value at 304.

After the maximum heartrate value at 304, the heartrate decreases till it reaches point 308 at about 28 cardiac cycles after the onset of the arousal at 300. At point 308, the heartrate remains substantially constant. At point 308, the continuous decrease of the heartrate has stopped. The recovery response phase is the phase in which the heartrate decreases after the maximum heartrate, so the recovery response phase signal 310 extends between the maximum heartrate value 304 and point 308. The recovery response phase signal 310 extends between cardiac cycles 5 and 28 after the onset of the arousal at 300.

FIG. 3 shows that in the recovery response phase signal 310, line 301 has higher value compared to line 302. Line 302 has returned to the same heart rate value as at the onset of the arousal, as indicated by dashed line 312, whereas line 301 is substantially above the dashed line 312. By comparing the recovery response phase signal 310 of a subject 101 with the dashed line 312, the likeliness of the subject 101 having either OSA or COMISA may be determined. A subject 101 having a heartrate according to line 301 likely has COMISA, whereas a subject 101 having a heartrate according to line 302 likely has OSA.

The peak response phase signal 306 represents a number of cardiac cycles in the range of 2-12 after the onset of the arousal at 300, i.e., 5 cardiac cycles. The recovery response phase signal 310 represents a number of cardiac cycles in the range of 5-25 after an end of the peak response phase, i.e., 23 cardiac cycles.

FIG. 4 depicts a change in heartrate of the subject 101 due to an arousal caused by an SDB event. The x-axis states the number of cardiac cycles, starting from the onset of the arousal at 300. The y-axis states the difference in heartbeats in beats per minute compared to the heartrate at the onset of the arousal at 300. Line 401 represents the averaged measured difference in heartrate of a COMISA patient caused by arousals caused by an SDB event. Line 402 represents the averaged measured difference in heartrate of an OSA patient caused by arousals caused by an SDB event.

For example, the subject 101 is using a PAP-device. A PAP-device provides continuous positive airway pressure to the subject 101 to help to prevent the upper airway of the subject 101 from collapsing. For example, the PAP-device has an airflow sensor configured to generate a signal representative of the airflow caused by the breathing of the subject 101. Because an SDB-event alters the breathing of the subject 101, the airflow sensor is configured to provide a signal representative of an SDB-event.

At the onset of the arousal at 300, both lines 401 and 402 increase similarly until they reach a maximum heartrate value at 404. The maximum heartrate value at 404 is reached after 5 cardiac cycles from the onset of the arousal at 300. The maximum heartrate value at 404 is about 7 beats per minute faster than the heartrate at the onset of the arousal at 300. Note that the maximum heart rate value at 404 is higher than the maximum heartrate value at 304 in FIG. 3. In FIG. 4, the peak response phase signal 306, which is representative of the peak response phase in which the heartrate increases after the arousal, starts at the onset of the arousal at 300 and ends at the maximum heartrate value at 404.

After the maximum heartrate value at 404, the heartrate decreases till it reaches point 408 at about 28 cardiac cycles after the onset of the arousal at 300. At point 408, the heartrate remains substantially constant. At point 408, the continuous decrease of the heartrate has stopped. The recovery response phase signal 310 extends between the maximum heartrate value 404 and point 408. The recovery response phase signal 310 extends between cardiac cycle 5 and 28 after the onset of the arousal at 300.

FIG. 4 shows that the recovery response phase signal 310 of line 401 has higher values compared to the recovery response phase signal 310 of line 402. Line 402 has returned to the same heart rate value as at the onset of the arousal, as indicated by dashed line 312, whereas line 401 is substantially above the dashed line 412. Further, FIG. 4 shows part of line 301 as a dashed line. At portion 406 of line 401, line 401 is substantially higher than line 301. So around cardiac cycles 10-15, an arousal caused by an SDB event causes a higher increase in the heartrate of a subject 101 with COMISA than an arousal not caused by an SDB-event. The heartrate of a subject with COMISA decreases more slowly after an arousal caused by an SDB-event than after an arousal not caused by an SDB-event. This insight may be used to determine whether a subject 101 has OSA or COMISA. A subject 101 having a heartrate according to line 401 likely has COMISA, whereas a subject 101 having a heartrate according to line 402 likely has OSA without insomnia.

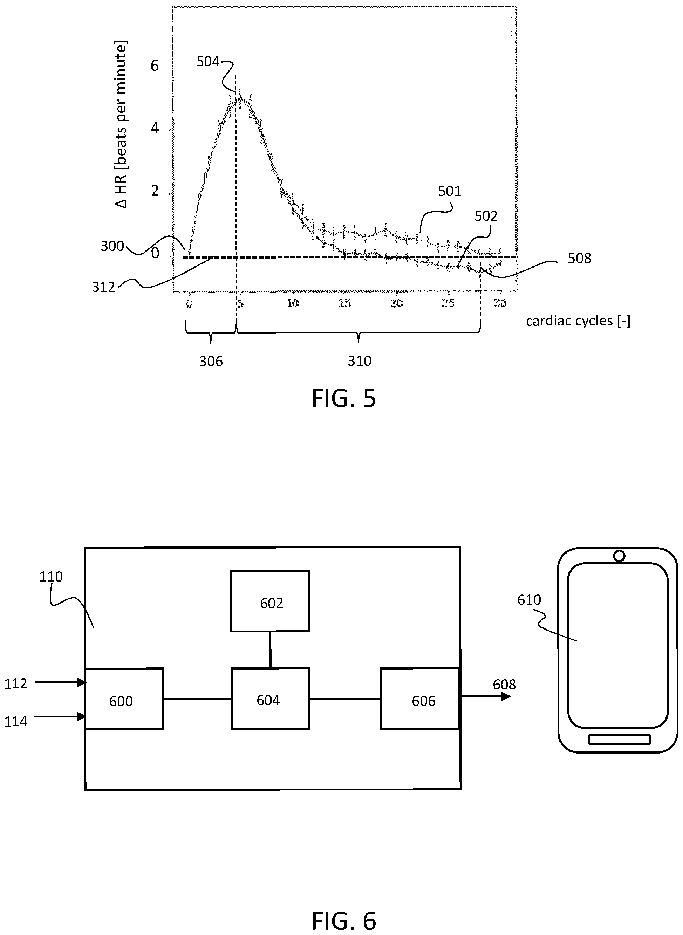

FIG. 5 depicts a change in heartrate of the subject 101 due to arousals not caused by an SDB-event. The x-axis states the number of cardiac cycles, starting from the onset of the arousal at 300. The y-axis states the difference in heartbeats in beats per minute compared to the heartrate at the onset of the arousal at 300. Line 501 represents the averaged measured difference in heartrate of a COMISA patient caused by arousals not caused by an SDB-event. Line 502 represents the averaged measured difference in heartrate of a OSA patient caused by arousals not caused by an SDB-event.

At the onset of the arousal at 300, both lines 501 and 502 increase similarly until they reach a maximum heartrate value at 504. The maximum heartrate value at 504 is reached after 5 cardiac cycles from the onset of the arousal at 300. The maximum heartrate value at 504 is about 5 beats per minute faster than the heartrate at the onset of the arousal at 300. The maximum heartrate value at 504 is lower than at 304 and 404. The peak response phase signal 306 starts at the onset of the arousal at 300 and ends at the maximum heartrate value at 504.

After the maximum heartrate value at 504, the heartrate decreases till it reaches point 508 at about 28 cardiac cycles after the onset of the arousal at 300. At point 508, the heartrate remains substantially constant. At point 508, the continuous decrease of the heartrate has stopped. The recovery response phase signal 310 extends between the maximum heartrate value 504 and point 508. The recovery response phase signal 310 extends between cardiac cycle 5 and 28 after the onset of the arousal at 300.

FIG. 5 shows that the recovery response phase signal 310 of line 501 has higher value compared to the recovery response phase signal 310 of line 502. Line 502 returns to the same heart rate value as at the onset of the arousal, as indicated by dashed line 312, after about 20 cardiac cycles, whereas line 501 returns to the dashed line 312 after about 28 cardiac cycles. Further, both lines 501 and 502 decrease quicker than lines 401 and 402. At cardiac cycle 10, lines 501 and 502 are around a difference in heartrate of 2, whereas lines 401 and 401 are well above 2 at cardiac cycle 10. This insight may be used to determine whether a subject 101 has OSA or COMISA. A subject 101 having a heartrate according to line 501 likely has COMISA, whereas a subject 101 having a heartrate according to line 502 likely has OSA without insomnia.

Any of the lines 301, 302, 401, 402, 501 and 502 may be used as a reference signal to compare with a measured heartrate of a subject 101. Any combinations of the lines 301, 302, 401, 402, 501 and 502 may be used as a reference signal and further reference signals.

In an example, the reference signal comprises an OSA-reference signal and a COMISA-reference signal. The OSA-reference signal is representative of a subject 101 having OSA, such as lines 302, 402 and 502. The COMISA-reference signal is representative of a subject 101 having COMISA, such as lines 301, 401 and 501. Determining the deviation comprises determining a first difference between the recovery response signal and the OSA-reference signal, and determining the deviation comprises determining a second difference between the recovery response signal and the COMISA-reference signal.

Instead of using the value of the heartrate prior to the arousal at 300, a pre-arousal phase signal may be determined in the heartrate signal 112. The reference signal is based the pre-arousal phase signal. The pre-arousal phase signal is representative of the heartbeat prior to an onset of the arousal. The pre-arousal phase signal is based on a number of cardiac cycles prior to the onset of the arousal, wherein the number is in the range of 2-12.

FIG. 6 depicts the processing unit 110 executing the computer-implemented method according to the first aspect of the invention. The processing unit 110 comprises an input terminal 600, a computer-readable storage medium 602, a processor 604 and an output terminal 606. The processor 604 is coupled to the input terminal 600, the computer-readable storage medium 602 and the output terminal 606. The input terminal 600 receives the arousal signal 114 and the heartrate signal 112 from the sensor system 102. The processor 604 receives the arousal signal 114 and the heartrate signal 112 from the input terminal 600. The computer-readable storage medium 602 stores a computer program product having instructions. When the processor 604 executes the instructions, the processing unit 110 carries out the computer-implemented method according to the first aspect of the invention. The processor 604 sends the deviation of the recovery response phase signal 310 relative to a reference signal to the output terminal 606. The output terminal 606 sends an output 608 based on the deviation to a user interface, such as a mobile phone 610.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. The computer program product may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A computer-implemented method comprising:
receiving an arousal signal (114) representative of an occurrence an arousal of a subject (101);
receiving a heartrate signal (112) representative of a heartrate of the subject (101);
determining a recovery response phase signal (310) in the heartrate signal (112),
wherein the recovery response phase signal (310) is representative of a recovery response phase in which the heartrate decreases after the arousal;
determining a deviation of the recovery response phase signal (310) relative to a reference signal.

2. The computer-implemented method of claim 1, comprising
receiving the arousal signal (114) from an arousal sensor (106);
receiving the heartrate signal (112) from a cardiac sensor (104);
wherein the arousal sensor (106) is configured to provide the arousal signal (1140 representative of the occurrence of the arousal,
wherein the cardiac sensor (104) is configured to provide the heartrate signal (112) representative of the heart rate.

3. The computer-implemented method of claim 1, comprising
receiving the arousal signal (114) from a cardiac sensor (104);
receiving the heartrate signal (112) from the cardiac sensor (104);
wherein the cardiac sensor (104) is configured to provide the arousal signal (114) representative of the occurrence of the arousal,
wherein the cardiac sensor (104) is configured to provide the heartrate signal (112) representative of the heart rate.

4. The computer-implemented method of any one of claims 2-3, comprising determining a peak response phase signal (306) in the heartrate signal (112),
wherein the peak response phase signal (306) is representative of a peak response phase in which the heartrate increases after the arousal,
wherein the recovery response phase signal (310) starts after the peak response phase signal (306).

5. The computer-implemented method of claim 4,
wherein the peak response phase signal (306) represents a number of cardiac cycles in the range of 2-12 after the arousal,
wherein the recovery response phase signal (310) represents a number of cardiac cycles in the range of 5-25 after an end of the peak response phase.

6. The computer-implemented method of any one of the preceding claims,
wherein determining the deviation comprises determining the deviation, in response to receiving an SDB signal representative of an occurrence of a Sleep Disorder Breathing (SDB) event of the subject (101).

7. The computer-implemented method of claim 6, wherein determining the deviation comprises determining the deviation of the recovery response signal relative to a further reference signal, in response to receiving a non-SDB signal representative of an absence of a Sleep Disorder Breathing (SDB) event of the subject (101).

8. The computer-implemented method of any one of the preceding claims, wherein the reference signal represents a recovery response phase of a subject (101) having one of Obstructive Sleep Apnea (OSA), Co-Morbid Insomnia and Sleep Apnea (COMISA) and insomnia.

9. The computer-implemented method of any one of the preceding claims,
wherein the reference signal comprises an OSA-reference signal and a COMISA-reference signal,
wherein the OSA-reference signal is representative of a subject (101) having OSA,
wherein the COMISA-reference signal is representative of a subject (101) having COMISA,
wherein determining the deviation comprises determining a first difference between the recovery response signal and the OSA-reference signal, and
wherein determining the deviation comprises determining a second difference between the recovery response signal and the COMISA-reference signal.

10. The computer-implemented method of any one of the preceding claims, wherein the reference signal represents a value of the heartrate prior to the arousal.

11. The computer-implemented method of any one of the preceding claims, comprising
determining a pre-arousal phase signal in the heartrate signal (112);
determining the reference signal based on the pre-arousal phase signal;
wherein the pre-arousal phase signal is representative of the heartbeat prior to an onset of the arousal.

12. The computer-implemented method of claim 11, wherein the pre-arousal phase signal is based on a number of cardiac cycles prior to the onset of the arousal, wherein the number is in the range of 2-12.

13. An apparatus (100) comprising
a sensor system (102); and
a processing unit (110) coupled to the sensor system (102);
wherein the sensor system (102) is configured to provide a heartrate signal (112) representative of a heartrate of a subject (101),
wherein the sensor system (102) is configured to provide an arousal signal (114) representative of an occurrence of an arousal of a subject (101),
wherein the processing unit (110) is configured to perform the computer-implemented method according to any one of the preceding claims.

14. A computer program product, comprising instructions which, when executed by a processing unit (110), cause the processing unit (110) to carry out the computer-implemented method of any one of clams 1-12.

15. A computer-readable storage medium comprising instructions which, when executed by a processing unit (110), cause the processing unit (110) to carry out the computer-implemented method of any one of clams 1-12.
